# EUROPEAN PATENT SPECIFICATION

(11) **EP 1 348 693 B1**
(45) Date of publication and mention of the grant of the patent: **12.09.2007**
(21) Application number: 02007068.6
(22) Date of filing: 27.03.2002
(51) Int. Cl.: C07C 303/22, C07C 309/88

(54) **A process for the preparation of diazonaphthoquinonesulfonylchlorides using diphosgene and triphosgene**
Verfahren zur Herstellung von Diazonaphthochinonsulfonylchloriden mit Diphosgen und Triphosgen
Procédé pour la préparation de chlorures d'acide diazonaphtoquinonesulfoniques utilisant diphosgène et triphosgène

(43) Date of publication of application: 01.10.2003
(73) Proprietor: COUNCIL OF SCIENTIFIC AND INDUSTRIAL RESEARCH, New Delhi 100 001 (IN)
(72) Inventor: Reddy, Vummadi Venkat, Hyderabad-500, 007, Andhra Pradesh (IN); Reddy, Maruthy Janaki Ram, Hyderabad-500, 007, Andhra Pradesh (IN); Rao, Vaidya Jayathirtha, Hyderabad-500, 007, Andhra Pradesh (IN)
(74) Representative: Henkel, Feiler & Hänzel

(56) References cited:
- E. SAUER, ET AL.: "Sulfochlorierung von 1,2-Naphthochinondiazid-(2) mit Chlorsulfonsäure" JOURNAL FÜR PRAKTISCHE CHEMIE, vol. 333, no. 3, 1991, pages 467-473, XP000925654 Leipzig, DE ISSN: 0021-8383
- R.C. REYNOLDS, ET AL.: "Synthesis of thymidine dimers containig internucleoside sulphonate and sulphonamide linkages" JOURNAL OF ORGANIC CHEMISTRY, vol. 57, no. 11, 22 May 1992 (1992-05-22), pages 2983-2985, XP002211505 American Chemical Society, Washington, DC, US ISSN: 0022-3263

## Description

The present invention relates to a process for the preparation of diazonaphthoquinonesulfonylchlorides, useful intermediates in electronic industry and dye industry. This research pertains to a method of preparation of diazonaphthoquinonesulfonylchlorides having formula **1-3** from corresponding diazonaphthoquinonesulfonic acid or its sodium salt, using diphosgene or triphosgene.

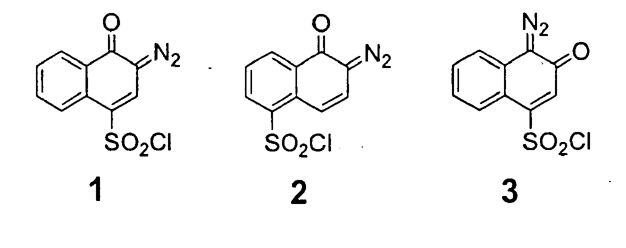

Our interest in preparing these diazonaphthoquinonesulfonylchlorides is to convert them in making various esters and which can be used in formulating the photoresists required for electronics industry.

### BACKGROUND OF THE INVENTION

There are a few methods of preparation of diazonaphthoquinonesulfonylchlorides reported in the literature and the same reports will be discussed below with merits and demerits.

The methods reported in prior art on the preparation of diazonaphthoquinonesulfonyl chlorides involves using chlorosulfonic acid and the corresponding diazonaphthoquinone sulfonic acid or its salt (CA, vol. 124, 32490e, PL 161,627, year 1993; CA vol. 64, 2033, USSR 173,756, year 1965; J.Prak.Chem., year 1991, vol. 333, p467). The main disadvantages of this method involves in using excess chlorosulfonic acid, reaction temperature and the evolution of gases like sulfurdioxide and hydrogenchloride.

Another method involves use of thionylchloride with dimethylformamide as catalyst with the corresponding diazonaphthoquinonesulfonic acid or its salt. This method also suffers the disadvantages like heating the reaction mixture, use of excess thionylchloride and evolution of sulfurdioxide and hydrogenchloride gases (CA, vol. 96, 34766b, Khim. Process, year 1981, p505 (Russ)).

Another method involves use of chlorosulfonic acid in combination with thionylchloride along with the corresponding diazonaphthoquinonesulfonic acid or its salt. The main disadvantages are the same as mentioned above (CA, vol. 105, 208620w, Ger (East) 272,511, year 1985 and DD 234, 000, year 1986; CA vol. 112, 178384x, Ger (East) DD 269,846, year 1989, Ger (East) 312,180, year 1988; CA, vol. 124, 302642u, JP 08,27,098, year 1996; CA, vol. 125, 170873d, RO 104,624, year 1994).

Yet another method involves is the use of phosgene (toxic gas) with the corresponding diazonaphthoquinonesulfonic acid or its salt. This method has the optimum temperature conditions but the greatest disadvantage is the use of toxic phosgene gas (CA, vol. 102, 113031d, JP 59,196,860, year 1984; CA, vol. 105, 60439w, EP 178,356, year 1986)

EP-A-0 524 634 discloses a process for preparing 1,2-naphthoquinonediazido-5-sulfonyl chloride which comprises reacting 1,2-naphthoquinonediazido-5-sulfonic acid or a salt thereof with phosgene in the presence of pyridine. According to this process, it is possible to prepare 1,2-naphthoquinonediazido-5-sulfonyl chloride in high yield with ease from 1,2-naphthoquinonediazido-5-sulfonic acid or a salt thereof.

### SUMMARY OF THE INVENTION

The present invention describes an altogether new process for the preparation of diazonaphthoquinonesulfonylchlorides by reacting the corresponding diazonaphthoquinone sulfonic acid or its salt either with diphosgene or triphosgene in presence of triethylamine base in dichloromethane solvent. Various solvents like, chloroform, 1,2-dichloroethane, benzene, toluene, acetonitrile, benzonitrile, nitrobenzene and others are also used. Dichloromethane as solvent was preferred. The temperature of the reaction was varied over -50 to +5°C and -40°C is the preferred temperature condition. The base is found to be essential for the reaction to be conducted and without the organic base there is no reaction occurs. The 2 mole equivalent ratio of base is the right combination found. The work up procedure and isolation of the product diazonaphthoquinonesulfonylchloride is very simple and rapid. After the reaction the organic base and the solvent can be recovered. After the isolation of the product, the remaining filtrate contains the unreacted diazonaphthoquinonesulfonic acid as judged by the UV-Visible absorption data. The products were characterized by the spectral data.

### DETAIL DESCRIPTION OF THE INVENTION

Accordingly the present invention provides a process for the preparation of diazonaphthoquinonesulfonylchlorides of formula 1-3, using diphosgene or triphosgene which comprises 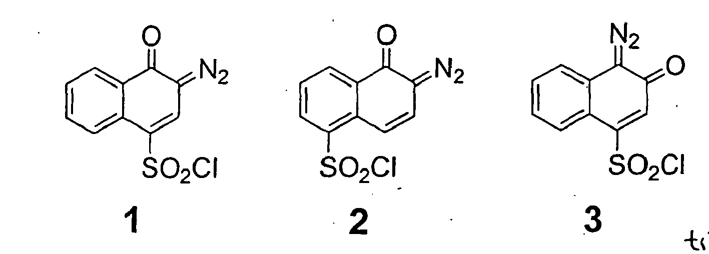 reacting diazonaphthoquinonesulfonicacid sodium salt with triethylamine in a molar ratio ranging between 1: 1.5 - 1: 2.5 in an organic solvent in the presence of diphosgene or triphosgene in a molar ratio of diazonaphthoquinonesulfonicacid sodium salt to diphosgene or triphosgene in the range of 1:1-1:1.5 ,at a temperature ranging from -50 °C to 5°C, for a period ranging from 40-90 min, subsequently increasing the temperature to 20-25°C, removing the solvent and base from the above said reaction mixture under vacuum to obtain the yellow powder product and re-precipitating the desired product in ice water.

In yet another embodiment the organic solvent used is selected from the group consisting of chloroform, 1,2-dichloroethane, benzene, toluene, acetonitrile, benzonitrile, nitrobenzene and dichloromethane.

In yet another embodiment the organic solvent used is dichloromethane.

In yet another embodiment the molar ratio of diazonaphthoquinonesulfonicacid sodium salt to organic base used is 1:2.

In yet another embodiment the molar ratio of diazonaphthoquinone sulfonicacid sodium salt to triphosgene or diphosgene used is 1:1.

In yet another embodiment A process as claimed in claim 1, wherein the reaction temperature used is -50 °C.

In yet another embodiment the diazonaphthoquinonesulfonicacid sodium salt used is selected from 2-Diazo-1-naphthoquinone-4-sulfonic acid sodium salt, 2-Diazo-1-naphthoquinone-5-sulfonic acid sodium salt and 1-Diazo-2-naphthoquinone-4-sulfonic acid sodium salt.

In yet another embodiment the diazonaphthoquinonesulfonul chloride obtained is selected from 2-Diazo-1-naphthoquinone-4-chloride of formula 1, 2-Diazo-1-naphthoquinone-5-sulfonulchloride of formula 2, and 1-Diazo-2-naphthoquinone-4-sulfonulchloridesulfonulchloride of formula 3.

The following examples are given by way of illustration of the present invention and therefore should not be construed to limit the scope of the present invention.

### Example 1:

2-Diazo-1-naphthoquinone-4-sulfonic acid sodium salt (2.72g; 0.01mol) was taken into 25 ml of dichloromethane and cooled to -50°C. Added triethylamine (2.02g; 0.02mol) to the above solution and maintained the temperature at -50°C.Then added diphosgene (2.18g; 0.011mol) in 15 ml dichloromethane very slowly and maintaining the temperature at -50°C with stirring over a period of 20min. The reaction mixture was stirred magnetically for 60 min at -50°C. Reaction mixture was brought to room temperature and then dichloromethane and triethylamine was removed under vacuum. The remaining yellow powder was poured into ice water after 5 min of holding it in ice water the precipitate formed was filtered, washed with ice water and dried in a vacuum desiccator. The dried 2-diazo-1-naphthoquinone-4-sulfonylchloride weight was 2.14g(0.0080mol) yield 80% m.p 138 -140°C. The 2-diazo-1-naphthoquinone-4-sulfonylchloride was characterized by UV-Visible absorption, ¹H-nmr and Mass spectrometry.

### Example 2:

2-Diazo-1-naphthoquinone-4-sulfonic acid sodium salt (10g; 0.037mol) was taken into 90 ml of dichloromethane and cooled to -50°C. Added triethylamine (7.4g; 0.073mol) to the above solution and maintained the temperature at -50°C.Then added diphosgene (7.35g; 0.037mol) in 15 ml dichloromethane very slowly and maintaining the temperature at -50°C with stirring over a period of 30min. The reaction mixture was stirred magnetically for 60 min at -50°C. Reaction mixture was brought to room temperature and then dichloromethane and triethylamine was removed under vacuum. The remaining yellow powder was poured into ice water, after 5 min of holding it in ice water the precipitate formed was filtered, washed with ice water and dried in a vacuum desiccator. The dried 2-diazo-1-naphthoquinone-4-sulfonylchloride weight was 7.93g (0.0296mol) yield 80%.

### Example 3:

2-Diazo-1-naphthoquinone-5-sulfonic acid sodium salt (2.72g; 0.01mol) was taken into 25 ml of dichloromethane and cooled to -50°C. Added triethylamine (2.02g; 0.02mol) to the above solution and maintained the temperature at -50°C.Then added diphosgene (2.18g; 0.011mol) in 15 ml dichloromethane very slowly and maintaining the temperature at -50°C with stirring over a period of 20min. The reaction mixture was stirred magnetically for 60 min at -50°C. Reaction mixture was brought to room temperature and then dichloromethane and triethylamine was removed under vacuum. The remaining yellow powder was poured into ice water, after 5 min of holding it in ice water the precipitate formed was filtered, washed with ice water and dried in a vacuum desiccator. The dried 2-diazo-1-naphthoquinone-5-sulfonylchloride weight was 2.14g(0.0080mol) yield 80% mp 135-138°C. The 2-diazo-1-naphthoquinone-5-sulfonylchloride was characterized by UV-Visible absorption, ¹H-nmr and Mass spectrometry.

### Example 4:

2-Diazo-1-naphthoquinone-5-sulfonic acid sodium salt (10g; 0.037mol) was taken into 90ml of dichloromethane and cooled to -50°C. Added triethylamine (7.4g; 0.073mol) to - the above solution and maintained the temperature at -50°C.Then added diphosgene (7.35g; 0.037mol) in 15ml dichloromethane very slowly and maintaining the temperature at -50°C with stirring over a period of 30min. The reaction mixture was stirred magnetically for 60 min at -50°C. Reaction mixture was brought to room temperature and then dichloromethane and triethylamine was removed under vacuum. The remaining yellow powder was poured into ice water after 5 min of holding it in ice water the precipitate formed was filtered, washed with ice water and dried in a vacuum desiccator. The dried 2-diazo-1-naphthoquinone-5-sulfonylchloride weight was 7.93g(0.0296mol) yield 80%.

**Example 5:** 1-Diazo-2-naphthoquinone-4-sulfonic acid sodium salt (2.72g; 0.01mol) was taken into 25 ml of dichloromethane and cooled to -50°C. Added triethylamine (2.02g; 0.02mol) to the above solution and maintained the temperature at -50°C. Then added diphosgene (2.18g; 0.011mol) in 15 ml dichloromethane very slowly and maintaining the temperature at -50°C with stirring over a period of 20 min. The reaction mixture was stirred magnetically for 60 min at -50°C. Reaction mixture was brought to room temperature and then dichloromethane and triethylamine was removed under vacuum. The remaining yellow powder was poured into ice water, after 5 min of holding it in ice water the precipitate formed was filtered, washed with ice water and dried in a vacuum desiccator. The dried 1-diazo-2-naphthoquinone-4-sulfonylchloride weight was 2.14 g(0.0080mol) yield 80%, m.p 138 -140°C. The 1-diazo-2-naphthoquinone-4-sulfonylchloride was characterized by UV-Visible absorption, ¹H-nmr and Mass spectrometry.

### Example 6:

2-Diazo-1-naphthoquinone-4-sulfonic acid sodium salt (2.72g; 0.01mol) was taken into 25 ml of dichloromethane and cooled to -50°C. Added triethylamine (2.02g; 0.02 mol) to the above solution and maintained the temperature at -50°C. Then added triphosgene (3.2g; 0.011 mol) in 15 ml dichloromethane very slowly and maintaining the temperature at -50°C with stirring over a period of 20 min. The reaction mixture was stirred magnetically for 60 min at -50°C. Reaction mixture was brought to room temperature and then dichloromethane and triethylamine were removed under vacuum. The remaining yellow powder was poured into ice water, after 5min of holding it in ice water the precipitate formed was filtered, washed with ice water and dried in a vacuum desiccator. The dried 2-diazo-1-naphthoquinone-4-sulfonylchloride weight was 2.14 g (0.0080mol) yield 80%.

### Example 7:

2-Diazo-1-naphthoquinone-5-sulfonic acid sodium salt (2.72g; 0.01 mol) was taken into 25 ml of dichloromethane and cooled to -50°C. Added triethylamine (2.02g; 0.02 mol) to the above solution and maintained the temperature at -50°C. Then added triphosgene (3.2g; 0.011 mol) in 15 ml dichloromethane very slowly and maintaining the temperature at -50°C with stirring over a period of 20 min. The reaction mixture was stirred magnetically for 60 min at -50°C. Reaction mixture was brought to room temperature and then dichloromethane and triethylamine were removed under vacuum. The remaining yellow powder was poured into ice water, after 5 min of holding it in ice water the precipitate formed was filtered, washed with ice water and dried in a vacuum desiccator. The dried 2-diazo-1-naphthoquinone-5-sulfonylchloride weight was 2.14 g (0.0080mol) yield 80%.

### Example 8:

1-Diazo-2-naphthoquinone-4-sulfonic acid sodium salt (2.72g; 0.01 mol) was taken into 25 ml of dichloromethane and cooled to -50°C. Added triethylamine (2.02g; 0.02 mol) to the above solution and maintained the temperature -50°C. Then added triphosgene (3.2g; 0.011 mol) in 15 ml dichloromethane very slowly and maintaining the temperature -50°C with stirring over a period of 20 min. The reaction mixture was stirred magnetically for 60 min at -50°C. Reaction mixture was brought to room temperature and then dichloromethane and triethylamine was removed under vacuum. The remaining yellow powder was poured into ice water, after 5 min of holding it in ice water the precipitate formed was filtered, washed with ice water and dried in a vacuum desiccator. The dried 1-diazo-2-naphthoquinone-4-sulfonylchloride weight was 2.14 g (0.0080mol) yield 80%.

### Advantages:

❖ The various advantages of this process methodology is given below:
❖ The main advantage of this method is that the very mild experimental conditions are well tuned and defined to get very good yields.
❖ The advantage of this method is that the reaction temperature is defined around -50°C to 0°C (preferably at -40°C).
❖ The advantage of this method is that the use of diphosgene/triphosgene novel reagents compared to toxic phosgene gas.
❖ The main advantage of this method is that there are no evolutions of corrosive gases like SO₂ and HCl making it environment friendly.
❖ The advantage of this method is that use of diphosgene/triphosgene (of 1.1 equivalents) is just optimum compare to other reported procedures, where phosgene, chlorosulfonicacid & thionylchloride employed are in excess.
❖ The advantage of this method is that it requires only two equivalents of triethylamine to conduct the reaction.
❖ The advantage of this method is that the organic base triethylamine used can be recovered

## Claims

1. A process for the preparation of diazonaphthoquinonesulfonyl chlorides of formula 1, 2, 3 using diphosgene or triphosgene which comprises 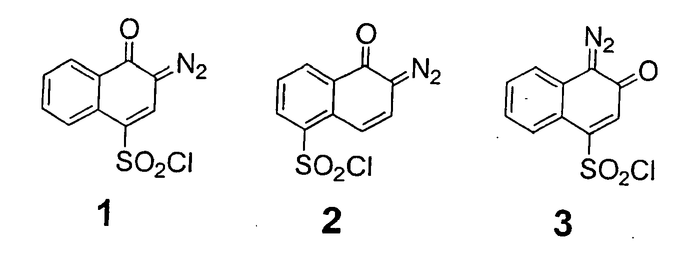 reacting diazonaphthoquinonesulfonic acid sodium salt with triethylamine in a molar ratio ranging between 1:1.5 - 1:2.5 in an organic solvent in the presence of diphosgene or triphosgene in a molar ratio of diazonaphthoquinonesulfonic acid sodium salt to diphosgene or triphosgene in the range of 1:1-1:1.5, at a temperature ranging from -50°C to 5°C, for a period ranging from 40 - 90 min, subsequently increasing the temperature to 20 - 25°C and removing the solvent and triethylamine from the above said reaction mixture under vacuum to obtain the yellow powder product and re-precipitating the desired product in ice water.

2. A process as claimed in claim 1, wherein the organic solvent used is selected from the group consisting of chloroform, 1,2-dichlorethane, benzene, toluene, acetonitrile, benzonitrile, nitrobenzene and dichloromethane.

3. A process as claimed in claim 2, wherein the organic solvent used is dichlormethane.

4. A process as claimed in claim 1, wherein the molar ratio of diazonaphthoquinonesulfonic acid sodium salt to triethylamine used is 1:2.

5. A process as claimed in claim 1, wherein the molar ratio of diazonaphthoquinonesulfonic acid sodium salt to triphosgene or diphosgene used is 1:1.

6. A process as claimed in claim 1, wherein the reaction temperature used is -50°C.

7. A process as claimed in claim 1, wherein the diazonaphthoquinonesulfonic acid sodium salt used is selected from 2-Diazo-1-naphthoquinone-4-sulfonic acid sodium salt, 2-Diazo-1-naphthoquinone-5-sulfonic acid sodium salt and 1-Diazo-2-naphthoquinone-4-sulfonic acid sodium salt.

## Patentansprüche

1. Verfahren zur Herstellung von Diazonaphthochinonsulfonylchloriden der Formeln 1, 2, 3 unter Verwendung von Diphosgen oder Triphosgen, 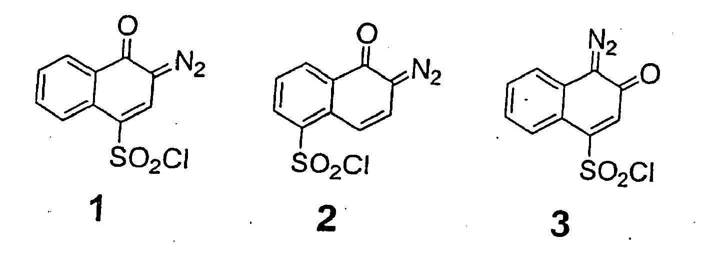 wobei das Verfahren die Umsetzung von Diazonaphthochinonsulfonsäurenatriumsalz mit Triethylamin in einem Molverhältnis im Bereich zwischen 1:1,5 und 1:2,5 in einem organischen Lösemittel in Gegenwart von Diphosgen oder Triphosgen in einem Molverhältnis von Diazonaphthochinonsulfonsäurenatriumsalz zu Diphosgen oder Triphosgen im Bereich von 1:1 bis 1:1,5 bei einer Temperatur im Bereich von -50 °C bis 5 °C über einen Zeitraum im Bereich von 40 - 90 min, das anschließende Erhöhen der Temperatur auf 20 - 25 °C und Entfernen des Lösemittels und von Triethylamin aus dem obigen Reaktionsgemisch unter Vakuum zur Gewinnung des gelben Pulverprodukts und erneutes Ausfällen des gewünschten Produkts in Eiswasser umfasst.

2. Verfahren gemäß Anspruch 1, wobei das verwendete organische Lösemittel aus der Gruppe von Chloroform, 1,2-Dichlorethan, Benzol, Toluol, Acetonitril, Benzonitril, Nitrobenzol und Dichlormethan ausgewählt ist.

3. Verfahren gemäß Anspruch 2, wobei das verwendete organische Lösemittel Dichlormethan ist.

4. Verfahren gemäß Anspruch 1, wobei das verwendete Molverhältnis von Diazonaphthochinonsulfonsäurenatriumsalz zu Triethylamin 1:2 beträgt.

5. Verfahren gemäß Anspruch 1, wobei das verwendete Molverhältnis von Diazonaphthochinonsulfonsäurenatriumsalz zu Triphosgen oder Diphosgen 1:1 beträgt.

6. Verfahren gemäß Anspruch 1, wobei die verwendete Reaktionstemperatur -50°C beträgt.

7. Verfahren gemäß Anspruch 1, wobei das verwendete Diazonaphthochinonsulfonsäurenatriumsalz aus 2-Diazo-1-naphthochinon-4-sulfonsäurenatriumsalz, 2-Diazo-1-naphthochinon-5-sulfonsäurenatriumsalz und 1-Diazo-2-naphthochinon-4-sulfonsäurenatriumsalz ausgewählt ist.

## Revendications

1. Procédé pour la préparation de chlorures de diazonaphtoquinonesulfonyle de formule 1, 2, 3 en utilisant du diphosgène ou du triphosgène, qui comprend 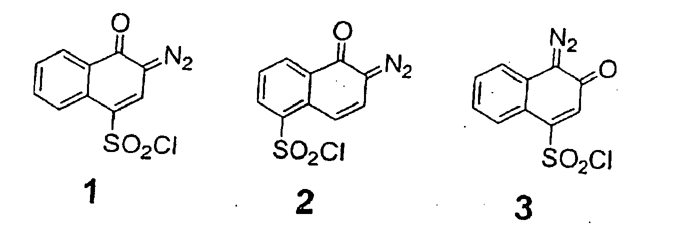 la réaction d'un sel sodique d'acide diazonaphtoquinonesulfonique avec de la triéthylamine dans un rapport molaire entre 1:1,5-1:2,5 dans un solvant organique en présence de diphosgène ou de triphosgène dans un rapport molaire du sel sodique d'acide diazonaphtoquinonesulfonique au disphogène ou triphosgène dans la gamme de 1:1 à 1:1,5, à une température allant de -50 °C à 5 °C, pendant une période allant de 40 - 90 min., puis l'augmentation de la température à 20-25 °C et l'élimination du solvant et de la triéthylamine à partir dudit mélange réactionnel précité sous vide pour obtenir le produit en poudre jaune et la reprécipitation du produit souhaité dans de l'eau glacée.

2. Procédé selon la revendication 1, dans lequel le solvant organique utilisé est choisi dans le groupe constitué par le chloroforme, le 1,2-dichloroéthane, le benzène, le toluène, l'acétonitrile, le benzonitrile, le nitrobenzène et le dichlorométhane.

3. Procédé selon la revendication 2, dans lequel le solvant organique utilisé est le dichlorométhane.

4. Procédé selon la revendication 1, dans lequel le rapport molaire du sel sodique d'acide diazonaphtoquinonesulfonique à la triéthylamine utilisé est de 1:2.

5. Procédé selon la revendication 1, dans lequel le rapport molaire du sel sodique d'acide diazonaphtoquinonesulfonique au triphosgène ou diphosgène utilisé est de 1:1.

6. Procédé selon la revendication 1, dans lequel la température de réaction utilisée est de -50 °C.

7. Procédé selon la revendication 1, dans lequel le sel sodique d'acide diazonaphtoquinonesulfonique utilisé est choisi parmi le sel sodique d'acide 2-diazo-1-naphtoquinone-4-sulfonique, le sel sodique d'acide 2-diazo-1-naphtoquinone-5-sulfonique et le sel sodique d'acide 1-diazo-2-naphtoquinone-4-sulfonique.
